# EUROPEAN PATENT APPLICATION

(11) **EP 1 188 441 A1**
(43) Date of publication of application: **20.03.2002**
(21) Application number: 00929901.7
(22) Date of filing: 29.05.2000
(51) Int. Cl.: A61K 31/663, A61K 9/08, A61K 9/10, A61K 9/46, A61K 47/26, A61K 47/18, A61K 47/20, A61K 47/10

(54) **ORAL PREPARATIONS OF ETIDRONATE DISODIUM**

(30) Priority: 02.06.1999 JP 15458699
(71) Applicant: Sumitomo Pharmaceuticals Company, Limited, Osaka-shi, Osaka 541-8510 (JP)
(72) Inventor: NISHII, Hiroyuki, Takatsuki-shi, Osaka 569-1029 (JP); OTODA, Kazuya, Takarazuka-shi, Hyogo 665-0877 (JP)
(74) Representative: Nargolwalla, Cyra
(86) International application number: JP0003449
(87) International publication number: WO0074685

(57) **Abstract**

Oral preparations containing a pharmaceutically effective amount of etidronate disodium and a sweetener, characterized in that these ingredients are administered in the state of totally or partly dissolved water. Since the characteristic taste of etidronate disodium has been relieved, these preparations can be administered even to patients with difficulty in swallowing and give little unpleasantness.

## Description

### Field of the Invention

This invention relates to oral preparations of disodium etidronate to prevent osteoabsorption in patients who have difficulty in swallowing.

### Background of the Invention

Etidronate disodium is a drug to control osteoabsorption in patients who have bone disease, including osteoporosis. There are cases of elderly female patients for whom etidronate disodium is administered for osteoporosis or who are at risk for the onset of osteoporosis, who also have difficulty in swallowing tablets. Currently etidronate disodium is administered to all patients as a tablet, and this is disadvantageous for some patients who have difficulty swallowing tablets. There is a possibility that these patients comprise a significant share of all patients using etidronate disodium. This is because osteoporosis, which is the main medicinal cause for use of etidronate disodium, occurs most commonly in elderly female patients. Furthermore, etidronate disodium tablets are directed to be administered together with large amounts of water, and there is concern that this leads to frequent urination.

Moreover, etidronate disodium has an unpleasant taste, with a unique combination of acidic, salty, and irritating flavors, which makes it difficult to develop other drug forms besides tablets. In the past, many methods to block or lessen the bitter taste of drugs have been disclosed. In particular, for orally administrated liquid drugs, the addition of non-water soluble medicinal polymers like ethyl cellulose or hydroxy-propyl-methyl cellulose phthalate (Patent S52-41214), the addition of acidic phospholipids or lysophospholipids (Patent H7-67552), or the addition of large amounts of citric acid (Patent H4-58452) have been disclosed. However, methods to lessen the unpleasant acidic, salty, and irritating taste seen with etidronate disodium are not commonly known.

Furthermore, etidronate disodium must be administered in extremely large doses of 200 mg to 1000 mg.

### Detailed Description of the Invention

The purpose of this invention is to provide a manufactured preparation that improves the unique taste of etidronate disodium, which must be administered in large doses, and makes administration easier in patients who have difficulty swallowing tablets, and is less unpleasant.

Rigorous investigations by the inventors have resulted in the discovery that the unpleasant acidic, salty, and irritating taste of etidronate disodium can be lessened by making an aqueous solution of etidronate disodium and adding a sweetener, to make a orally administered manufactured preparation that can easily be administered to elderly females, thus completing this invention. In this document, an orally administered manufactured preparation refers to a drug form that is administered in a condition where some or all of the medicinally effective quantity of etidronate disodium is dissolved in water. Specifically, these forms include liquids, syrups, dissolve-before-use dry syrups, gels, and effervescing materials. These orally administered forms of etidronate disodium preparations have the advantage that they are easily administered and readily accepted by patients who have difficulty in swallowing solid orally administered preparations. Furthermore, because the large volume of water required when conventional tablets are taken is not absolutely necessary, the risk of frequent urination caused by that administration method is relieved.

To summarize, this invention is as shown below.
(1) An oral preparation characterized by the fact that it contains medicinally effective quantities of etidronate disodium and a sweetener and that it is administered in a form totally or partially dissolved in water.
(2) The oral preparation shown in (1), where the preparation is a liquid, syrup, dry syrup, gel, or effervescing compound.
(3) The oral preparation shown in (1) and (2), which contains between 0.1 and 50 w/v% etidronate disodium.
(4) The oral preparation shown in any of(1) through (3), where the sweetener is selected from any of or a mixture of two or more of lactose, glucose, fructose, maltose, sucrose, trehalose, mannitol, xylitol, erythritol, sorbitol, maltitol, aspartame, saccharine, sodium saccharine, stevia, thaumatin, and glycerin.
(5) The oral preparation shown in any of (1) through (3), where the sweetener is either erythritol or glycerin.
(6) The oral preparation shown in any of (1) through (5) where the pH, if the oral preparation is a liquid, or, if the oral preparation is in a form other than a liquid, the pH when the preparation is dissolved or dispersed in between 2 and 10 times (w/w) the quantity of water, is between 3 and 8.
(7) The oral preparation shown in any of (1) through (6), characterized by the fact that it also contains a fragrance.
(8) The oral preparation shown in (1), which is a liquid with the following composition:

| | |
|---|---|
| etidronate disodium | 10 - 1000 mg |
| erythritol | 10 - 1000 mg |
| purified water | up to a total volume of 10 ml. |

(9) The oral preparation shown in (1), which is a liquid with the following composition:

| | |
|---|---|
| etidronate disodium | 10 - 1000 mg |
| erythritol | 10 - 1000 mg |
| glycerin | 10 -1000 mg |
| lemon | 10 - 1000 mg |
| purified water | up to a total volume of 10 ml. |

[10] The oral preparation shown in (1), which is a liquid with the following composition:

| | |
|---|---|
| etidronate disodium | 10 - 1000 mg |
| erythritol | 10 - 1000 mg |
| citric acid | 1 - 15 mg |
| sodium citrate | 5 - 50 mg |
| saccharine | 0.1 - 20 mg |
| plum flavor | 0.1 - 10 mg |
| purified water | up to a total volume of 10 ml. |

[11] The oral preparation shown in (1), which is a dry syrup with the following composition:

| | |
|---|---|
| etidronate disodium | 10 - 200 mg |
| erythritol | 10 - 1000 mg |

[12] The oral preparation shown in (1), which is a gel with the following composition:

| | |
|---|---|
| etidronate disodium | 10 - 1000 mg |
| erythritol | 10 - 2000 mg |
| gelatin | 5 - 100 mg |
| plum flavor | 0.1 - 10 mg |
| purified water | up to a total volume of 10 ml. |

Examples of sweeteners for this invention include lactose, glucose, fructose, maltose, sucrose, aspartame, saccharine, sodium saccharine, stevia, thaumatin, trehalose, mannitol, erythritol, sorbitol, xylitol, maltitol, and glycerin, or mixtures of these. Preferred sweeteners include sucrose, sorbitol, xylitol, erythritol and glycerin, but erythritol and glycerin are especially preferred. The weight ratio of etidronate disodium to sweetener is not specifically restricted, but a range of 1:0.01 to 1:50 is suggested, with a preferred range of 1:0.1 to 1:10.

The pH of the preparation is between 2 and 9, preferably between 3 and 8. The pH of the preparation as defined here is the pH of the liquid if the preparation is a liquid, and if the preparation is in a form other than liquid, the pH when the preparation is dissolved or dispersed in ten parts of water (w/w) for each part of preparation. If there is a need to adjust the pH of the preparation, a commonly used pH modifier may be added. Hydrochloric acid and sodium hydroxide are suggested as possible pH modifiers.

If the pH shifts particularly during storage, a sufficient quantity of buffer may be added to maintain the designated pH. Suggested buffers include malic acid, citric acid, tartaric acid, ascorbic acid, succinic acid, fumaric acid, maleic acid, gluconic acid, glucuronic acid, and phosphoric acid, or any mixture of these. Preferred buffers include malic acid, citric acid, tartaric acid, and phosphoric acid, or a salt of any of these compounds, but citric acid and salts thereof are particularly favorable. Fragrances may be added to further improve the sensation associated with the dosage. Suggested fragrances and flavorings include lemon, orange, grapefruit, pineapple, fruit, yogurt, plum, etc.

Besides the above, non-poisonous non-reactive additives that are commonly used in the field of pharmaceuticals may be added to the oral preparation of this invention. These additives do not have an impact on the actual effectiveness of this invention, and common medical product additives may be added. These additives may include fillers such as cornstarch, potato starch, talc, kaolin, calcium hydrogen phosphate, calcium sulfate, calcium carbonate, and crystallized cellulose, lubricants like magnesium stearate, magnesium stearate (sic), and calcium stearate, disintegrators like calcium carboxy-methyl cellulose and low substitution hydroxy-methyl cellulose, and binders like hydroxy-propyl cellulose, hyroxy-propyl-methyl cellulose, polyvinyl pyrrolidone, gelatin, methyl cellulose, gum-arabic powder, and poly-vinyl alcohol, as well as other coloring additives, spices, absorbents, preservatives, stabilizers, wetting agents, and anti-static agents.

The form of the oral preparation of this invention is not restricted, but liquid, syrup, dry syrup, gel, and effervescing tablets are preferred. The production methods normally used for each product form may be used, and there are no restrictions in particular.

The amount of etidronate disodium used in the oral preparations associated with this invention is in the range of 0.05 to 90 w/v%, and a level of 0.1 to 50 w/v% is suggested as being particularly favorable. Dosages in the range of 100 mg to 2000 mg per day of active ingredient are suggested.

Using the methods of this invention, patients, in particular post menopausal women, can be treated with osteogenically effective quantities of etidronate disodium, and osteoabsorption, which requires this type of treatment, can be blocked. The need for this type of treatment occurs locally with such conditions as bone fractures, artificial joints, and bone loss, as well as with systemic bone disease like osteoporosis, osteoarthritis, Paget's disease, osteomalacia, recurrent myeloma and other forms of cancer, steroid treatments, and age-related bone loss.

### Preferred Embodiments

Preferred embodiments and comparison cases have been given below and explained in further detail, but this invention is not necessarily restricted to these cases.

### Preferred Embodiment 1

| Etidronate disodium solution | |
|---|---|
| Etidronate disodium | 200 mg |
| Erythritol | 250 mg |
| Purified water | 2 ml |
| Dissolve the etidronate disodium and erythritol in purified water to make an etidronate disodium solution. | |

### Preferred Embodiment 2

| Etidronate disodium solution | |
|---|---|
| Etidronate disodium | 200 mg |
| Erythritol | 200 mg |
| Glycerin | 200 mg |
| Yogurt flavoring | 5 mg |
| Purified water | 2 ml |
| Dissolve the etidronate disodium, erythritol, and glycerin in purified water to make an etidronate disodium solution. | |

### Preferred Embodiment 3

| Etidronate disodium solution | |
|---|---|
| Etidronate disodium | 200 mg |
| Erythritol | 250 mg |
| Plum flavoring | 1 mg |
| Purified water | 1 ml |
| Dissolve or disperse the etidronate disodium, erythritol, and plum flavor in purified water to make an etidronate disodium solution. | |

### Preferred Embodiment 4

| Etidronate disodium syrup | |
|---|---|
| Etidronate disodium | 200 mg |
| Purified sucrose | 1000 mg |
| Grapefruit flavoring | 2 mg |
| Purified water | 2 ml |
| Dissolve or disperse the etidronate disodium, pure sucrose, and grapefruit flavoring in purified water to make an etidronate disodium syrup. | |

### Preferred Embodiment 5

| Etidronate disodium solution | |
|---|---|
| Etidronate disodium | 200 mg |
| Glycerin | 200 mg |
| Citric acid | 6 mg |
| Sodium citrate | 20 mg |
| Purified water | 1 ml |
| Dissolve the etidronate disodium, glycerin, citric acid and sodium citrate in purified water to make an etidronate disodium solution. | |

### Preferred Embodiment 6

| Etidronate disodium dry syrup | |
|---|---|
| Etidronate disodium | 200 mg |
| Erythritol | 800 mg |
| Mix the etidronate disodium and the erythritol together to make an etidronate disodium dry syrup. | |

### Preferred Embodiment 7

| Etidronate disodium gel | |
|---|---|
| Etidronate disodium | 200 mg |
| Gelatin | 100 mg |
| Erythritol | 800 mg |
| Plum flavoring | 10 mg |
| Purified water | 10 ml |
| Dissolve the gelatin in purified water heated to above 80° C. After dissolving or dispersing the etidronate disodium, erythritol, and plum flavoring in this solution, cool to make an etidronate disodium gel. | |

### Preferred Embodiment 8

| Etidronate disodium solution | |
|---|---|
| Etidronate disodium | 200 mg |
| Erythritol | 313 mg |
| Glycerin | 250 mg |
| Lemon essence | 0.5 mg |
| Sodium hydroxide | As appropriate |
| Purified water | As appropriate |
| After dissolving the etidronate disodium, erythritol, glycerin, methyl para-oxybenzoate, propyl para-oxybenzoate, and lemon essence in the purified water, add sodium hydroxide to adjust the pH to 5.5, to make a 5 ml etidronate disodium solution. | |

### Comparison Example 1

| Etidronate disodium solution | |
|---|---|
| Etidronate disodium | 200 mg |
| Purified water | 2 ml |
| Dissolve the etidronate disodium in the purified water to make an etidronate disodium solution. | |

### Test Example

### Taste Test

A taste test was performed by a three-member panel using the etidronate disodium solutions obtained from Preferred Embodiments 1 and 2 and Comparison Example 1. The judgments for the taste test are described in three levels shown below.
Satisfactory: Absolutely no unpleasant taste detected, and the sensation of the dosage was satisfactory.
Normal: A slight unpleasant taste, and sensation of the dosage was normal.
Poor: The taste was unpleasant, or the sensation of the dosage was poor.

The results are shown in Table 1.

**Table 1**

| Panel member | Preferred embodiment 1 | Preferred embodiment 2 | Preferred embodiment 8 | Comparison example 1 |
|---|---|---|---|---|
| 1 | Satisfactory | Satisfactory | Satisfactory | Poor |
| 2 | Normal | Satisfactory | Satisfactory | Poor |
| 3 | Satisfactory | Satisfactory | Satisfactory | Poor |

### Industrial Field of Use

Through this invention, oral preparations of etidronate disodium can be provided where the unpleasant bitter and irritating taste has been improved and sensation of the dosage is excellent. Therefore, adults, and in particular, elderly female patients who are most susceptible to osteoporosis, can easily take sufficient quantities of etidronate disodium.

## Claims

1. An oral preparation, **characterized by** the fact that it contains medicinally effective quantities of etidronate disodium and a sweetener and that it is administered in a form totally or partially dissolved in water.

2. The oral preparation shown in Claim 1, where the preparation is either a liquid, syrup, dry syrup, gel, or effervescing compound.

3. The oral preparation shown in Claim 1 and Claim 2, which contains between 0.1 and 50 w/v% etidronate disodium.

4. The oral preparation shown in any of Claim 1 through Claim 3, where the sweetener is selected from any of or a mixture of 2 or more of lactose, glucose, fructose, maltose, sucrose, trehalose, mannitol, xylitol, erythritol, sorbitol, maltitol, aspartame, saccharin, sodium saccharine, stevia, thaumatin, and glycerin.

5. The oral preparation shown in any of Claim 1 through Claim 3, where the sweetener is either erythritol or glycerin.

6. The oral preparation shown in any of Claim 1 through Claim 5, where the pH, if the oral preparation is a liquid, or, if the oral preparation is in a form other than a liquid, the pH when the preparation is dissolved or dispersed in between 2 and 10 times (w/w) the quantity of water, is between 3 and 8.

7. The oral preparation shown in any of Claim 1 through Claim 6, which is **characterized by** the fact that it also contains a fragrance.

8. The oral preparation shown in Claim 1, which is a liquid with the following composition:
| | |
|---|---|
| etidronate disodium | 10 - 1000 mg |
| erythritol | 10 - 1000 mg |
| purified water | up to a total volume of 10 ml. |

9. The oral preparation shown in Claim 1, which is a liquid with the following composition:
| | |
|---|---|
| etidronate disodium | 10 - 1000 mg |
| erythritol | 10 - 1000 mg |
| glycerin | 10 - 1000 mg |
| lemon | 10 - 1000 mg |
| purified water | up to a total volume of 10 ml. |

10. The oral preparation shown in Claim 1, which is a liquid with the following composition:
| | |
|---|---|
| etidronate disodium | 10 - 1000 mg |
| erythritol | 10 - 1000 mg |
| citric acid | 1 - 15 mg |
| sodium citrate | 5 - 50 mg |
| saccharine | 0.1 - 20 mg |
| plum flavor | 0.1 - 10 mg |
| purified water | up to a total volume of 10 ml. |

11. The oral preparation shown in Claim 1, which is a dry syrup with the following composition:
| | |
|---|---|
| etidronate disodium | 10 - 200 mg |
| erythritol | 10 - 1000 mg |

12. The oral preparation shown in Claim 1, which is a gel with the following composition:
| | |
|---|---|
| etidronate disodium | 10 - 1000 mg |
| erythritol | 10 - 2000 mg |
| gelatin | 5 - 100 mg |
| plum flavor | 0.1 - 10 mg |
| purified water | up to a total volume of 10 ml. |
